(19) Europäisches Patentamt
European Patent Office
Office européen des brevets

(11) Veröffentlichungsnummer : **0 012 294**
**B1**

# (12) EUROPÄISCHE PATENTSCHRIFT

(45) Veröffentlichungstag der Patentschrift : **16.02.83**

(51) Int. Cl.$^3$ : **C 07 D251/50**

(21) Anmeldenummer : **79104806.9**

(22) Anmeldetag : **01.12.79**

## (54) Quartäre Reaktivverbindungen, ihre Herstellung und ihre Verwendung zur Affinitätserhöhung von anionischen Farbstoffen zu stickstoff- oder hydroxylgruppenhaltigen Fasern.

(30) Priorität : **14.12.78 DE 2853881**

(43) Veröffentlichungstag der Anmeldung : **25.06.80 Patentblatt 80/13**

(45) Bekanntmachung des Hinweises auf die Patenterteilung : **16.02.83 Patentblatt 83/07**

(84) Benannte Vertragsstaaten : **CH DE FR GB**

(56) Entgegenhaltungen :
**DE A 2 626 495**
**DE A 2 726 432**
**DE A 2 726 433**
**GB A 1 166 741**
**Die Akte enthält technische Angaben, die nach dem Eingang der Anmeldung eingereicht wurden und die nicht in dieser Patentschrift enthalten sind.**

(73) Patentinhaber : **BAYER AG**
**Zentralbereich Patente, Marken und Lizenzen**
**D-5090 Leverkusen 1, Bayerwerk (DE)**

(72) Erfinder : **Berger-Lohr, Bernd, Dr.**
**Heymannstrasse 63**
**D-5090 Leverkusen 1 (DE)**
Erfinder : **Schündehütte, Karl Heinz, Dr.**
**Kllef 75**
**D-5090 Leverkusen 3 (DE)**
Erfinder : **Söll, Manfred, Dr.**
**Dillinger Strasse 23**
**D-5090 Leverkusen 1 (DE)**

**EP 0 012 294 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

# Quartäre Reaktivverbindungen, ihre Herstellung und ihre Verwendung zur Affinitätserhöhung von anionischen Farbstoffen zu stickstoff- oder hydroxylgruppenhaltigen Fasern

Die vorliegende Erfindung betrifft quartäre Verbindungen der Formel (I)

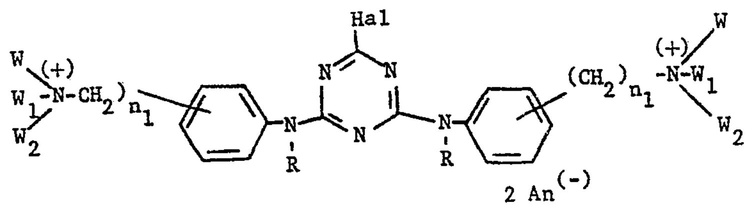

(I)

worin

Hal für Fluor oder Chlor,

R für Wasserstoff oder $C_1$-$C_4$-Alkyl,

$n_1$ für 1 oder 2,

W, $W_1$ und $W_2$ unabhängig voneinander für $C_1$-$C_6$-Alkyl das durch

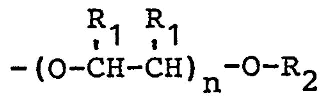

substituiert sein kann, wobei beide $R_1$ für Wasserstoff stehen oder 1 $R_1$ für Wasserstoff und das andere für Methyl steht $R_2$ Wasserstoff oder $C_1$-$C_4$-Alkyl ist und,

W außerdem für Benzyl oder

$W_1$ und $W_2$ gemeinsam mit dem Stickstoffatom für Pyrrolidin, Piperidin, Morpholin oder Piperazin oder

W, $W_1$ und $W_2$ gemeinsam mit dem Stickstoffatom für Pyridin und

$An^{(-)}$ für ein Anion stehen.

Besonders bevorzugt werden Verbindungen der Formel (I) mit

R = Wasserstoff oder Methyl,

W, $W_1$ und $W_2$ = Methyl, Ethyl oder Hydroxyethyl,

und $n_1 = 1$

Die Herstellung der Verbindungen der Formel (I) kann nach an sich bekannten Methoden z. B. nach den Verfahren 1 und 2 erfolgen :

1.

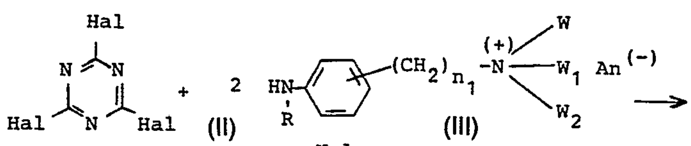

(II) (III) (I)

2.

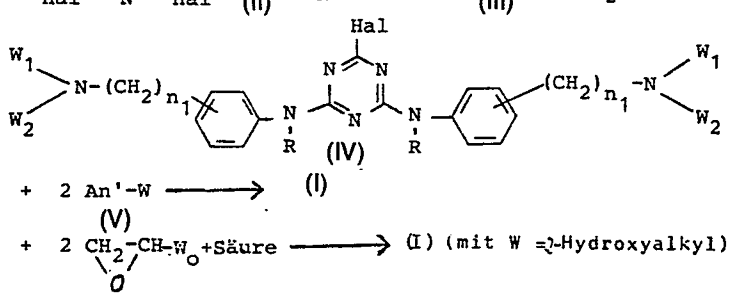


(IV) (V) (I) (VI)

worin

An' für einen als Anion $An^{(-)}$ abspaltbaren Rest und

$W_0$ für Wasserstoff oder Alkyl, das 2 Kohlenstoffatome weniger als W hat, stehen,

und die übrigen Symbole die Bedeutung der Formel (I) haben.

Die Umsetzung nach 1 kann in wäßriger oder wäßrig-organischer Lösung, vorzugsweise in neutraler oder schwach saurer Lösung bei Temperaturen zwischen 0 und 80 °C, vorzugsweise 0-40 °C erfolgen. Die während der Reaktion entstehende Säure H-Hal wird beispielsweise mit Bicarbonat, Soda oder Natronlauge neutralisiert. Die Verbindungen der Formel-(I) werden als feste Substanzen oder in Pastenform aus der Reaktionslösung isoliert. Es ist jedoch nicht erforderlich, die Verbindungen I aus dem Reaktionsmedium zu isolieren. Sie können beispielsweise auch in Form der bei der Herstellung

erhaltenen Lösungen angewendet werden. Zur Verbesserung der Stabilität können dem Reaktionsmedium vor, während oder nach der Reaktion Puffersubstanzen wie Phosphate, Maleinate, Acetate, Carbonate oder Citrate zugesetzt werden. Als Beispiele für Verbindungen der Formel (III) seien aufgeführt :

1. 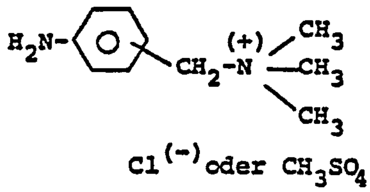

$Cl^{(-)}$ oder $CH_3SO_4^{(-)}$

2. 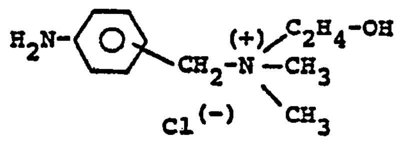

$Cl^{(-)}$

3. 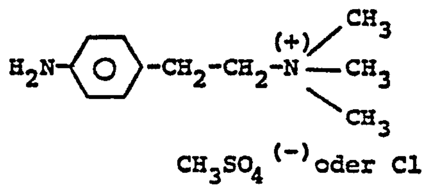

$CH_3SO_4^{(-)}$ oder $Cl^{(-)}$

4. 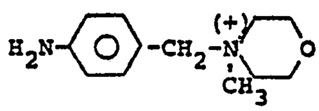

$CH_3-SO_4^{(-)}$

5. 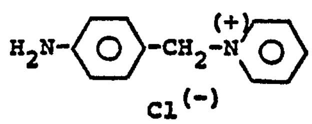

$Cl^{(-)}$

6. 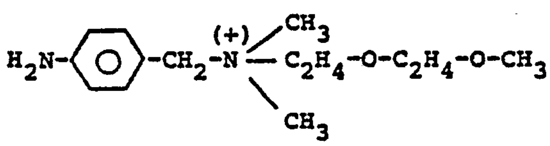

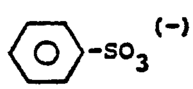

Als Verbindungen II seien hier auszugsweise erwähnt : 2,4,6-Trifluortriazin, 2-Chlor-4,6-difluor-triazin, 2,4-Dichlor-6-fluor-triazin und Cyanurchlorid.

Als Quaternierungsmittel (V) und (VI) seien genannt : Alkylhalogenide, Benzylhalogenide, Dialkylsulfate, Alkylester von Arylsulfonsäuren sowie andere Ester starker Mineralsäuren und organischer Sulfonsäuren von vorzugsweise niedrigen Alkoholen. In Gegenwart von Säuren können auch Epoxide als Alkylierungsmittel verwendet werden. Die quaternierenden Mittel können weiter substituiert sein. Beispiele sind : Dimethylsulfat, Diethylsulfat, Methylchlorid, Methylbromid, Methyljodid, Methylsulfat, Ethylbromid, n-Propylbromid, n-Butylbromid, Benzylchlorid, Ethylenoxid und Propylenoxid.

Man kann die erfindungsgemäß zu verwendenden Verbindungen der Formel (I) in einem Vorbehandlungsprozeß auf das zu färbende Fasermaterial aufbringen, oder man wendet sie einbadig mit den anionischen Farbstoffen zusammen an. Man kann sie auch in einem Nachbehandlungsprozeß auf fertige Färbungen und Drucke aufbringen. Im Falle einer Vorbehandlung wird das mit den Verbindungen der Formel (I) behandelte Fasermaterial anschließend aus langer Flotte in der für die jeweiligen Farbstoffe üblichen Weise gefärbt. Man kann das Material aber auch aus kurzer Flotte färben.

Eine Vorbehandlung erfolgt durch Imprägnieren oder Bedrucken mit wäßrigen Klotzflotten bzw.

Druckpasten, die neben den Verbindungen (I) in Mengen von 20-150 g vorzugsweise 40-100 g, pro Liter Klotzflotte bzw. Druckpaste das zur chemischen Umsetzung mit dem Fasermaterial notwendig Alkali, z. B. Natriumbicarbonat, Natriumhydroxid oder vorzugsweise Natriumcarbonat, enthält. Das so imprägnierte Fasermaterial wird auf eine Flottenaufnahme von 60-100 %, vorzugsweise 70-90 %, des Fasergewichts abgepreßt und mit oder ohne Zwischentrocknung einer Hitzebehandlung unterzogen ; dies kann durch kurzfristiges Dämpgen, z. B. 3 bis 10 Minuten Dampfbehandlung bei 102 bis 120 °C, oder durch kurzfristige Trockenhitzebehandlung, z. B. 2 bis 10 Minuten bei 120 bis 150 °C erfolgen ; bei dieser Hitzebehandlung erfolgt die Reaktion der Verbindungen (I) mit dem Fasermaterial unter Ausbildung einer chemischen Bindung. Die Reaktion mit dem Fasermaterial kann aber auch in einem Kaltverweilverfahren erfolgen, indem man das imprägnierte und abgepreßte Material aufrollt und bei Zimmertemperatur 4 bis 24 Stunden bzw. bei Raumtemperatur aufbewahrt. Durch Umwickeln mit wasserundurchlässigem Material muß dabei Sorge getragen werden, daß kein Wasser verdampfen kann.

Die Verweilzeit kann durch Erhöhung der Verweiltemperatur von Raumtemperatur auf 60-80 °C auf eine bis vier Stunden vermindert werden.

Bei der einbadigen Anwendung der Verbindungen (I) zusammen mit Farbstoffen stellt man Klotzflotten bzw. Druckpasten her, die neben den Verbindungen (I) in Mengen von 20-150 g, vorzugsweise 40-100 g je Liter Klotzflotte bzw. je kg Druckpaste, die anionischen Farbstoffe und noch die in der Färberei üblichen Klotzhilfsmittel und Netzmittel, die im Textildruck üblichen Verdickungsmittel und Reduktionsmittel sowie das notwendige alkalische Mittel, z. B. Natriumbicarbonat, Natriumcarbonat oder Natriumhydroxid enthalten. Die anschließende Fixierung erfolgt in der üblichen Weise durch eine Dampf-, Trockenhitze- oder Kaltverweilbehandlung.

Zur Nachbehandlung fertiger Färbungen oder Drucke setzt man die Verbindungen (I) in Mengen von 0,1-8 %, vorzugsweise 1-5 %, bezogen auf das Substrat in wäßriger Lösung ein. Die Lösungen enthalten 1-5 ml/l Natriumhydroxid (50 %ig). In diesen Lösungen werden die fertigen Färbungen 10-60 Minuten lang bei Temperaturen von 20-80 °C vorzugsweise 20-40 °C, behandelt.

Zur Nachbehandlung kann man auch Klotzflotten herstellen, die neben den Verbindungen der Zusammensetzung (I) in Mengen von 20-150 g, vorzugsweise 40-100 g je Liter Klotzflotte, noch die in der Färberei üblichen Klotzhilfsmittel und Netzmittel sowie das notwendige alkalische Mittel, z. B. Natriumbicarbonat, Natriumcarbonat oder Natriumhydroxid enthalten. Hier erfolgt die anschließende Fixierung durch eine Dampf-, Trockenhitze- oder Kaltverweilbehandlung.

Mit Hilfe der erfindungsgemäßen Verbindungen (I) erzielt man eine überraschend große Verbesserung der Farbechteiten, insbesondere der Wasser-, Schweiß- und Waschechtheiten, gegenüber den Farbechteiten üblicher Färbungen und Drucke mit anionischen Farbstoffen. Es werden außerdem tiefere Färbungen und Drucke erzielt, da Spül- und Seifnachbehandlung zu keiner Ablösung der Farbstoffe vom Fasermaterial führen, wie es sonst bei nicht behandelten Färbungen und Drucken der Fall ist.

Die Verwendung der Verbindungen (I) erfolgt auf Textilmaterial aus natürlicher Cellulose, wie Baumwolle oder Leinen, oder regenerierte Cellulose wie Zellwolle, Rayon oder Modulfasern ; diese Fasermaterialien können sowohl allein als auch in Mischungen mit synthetischen Fasermaterialien z. B. solchen aus Polyester, Polyamid oder Polyacrylnitril vorliegen.

Verbindungen der Zusammensetzung (I) können zur Vor-, Einbad- und Nachbehandlung von Färbungen und Drucken mit solchen Farbstoffen dienen, wie sie z. B. im Colour Index, 3. Auflage (1971), Bd. 1 auf den Seiten 1001-1561 als saure Farbstoffe und Bd. 2 auf den Seiten 2005-2477 als Direktfarbstoffe aufgeführt sind.

Im Vergleich zu den bekannten kationaktiven Nachbehandlungsmitteln für Direktfarbstoffe bestehen Vorteile der Verbindungen der Zusammensetzung (I) darin, daß die mit ihnen vor-, einbadig- bzw. nachbehandelten Färbungen bedeutend verbesserte Waschechtheiten auch bei erhöhten Waschtemperaturen, z. B. bei 60 °C, und sogar bei 95 °C, ergeben. Die Lichtechtheiten werden nicht negativ beeinflußt, und es sind nur geringfügige oder keine Farbtonänderungen feststellbar.

Überraschenderweise zeigt es sich, daß die Verbindungen (I) sogar gegenüber chemisch naheliegenden Verbindungen Vorteile zeigen. Aus DE-A-2 626 495, DE-A-2 726 432 und DE-A-2 726 433 sind Triazinverbindungen bekannt, deren quartäre Gruppe direkt mit dem Benzolring verbunden ist, d. h. Verbindungen der Formel (I), in der $n_1$ für O steht. Gegenüber entsprechenden Fluortriazinen verbessern die neuen Verbindungen die Waschechtheiten in einem höheren Maße, und gegenüber entsprechenden Chlortriazinen zeigen die neuen Verbindungen eine erhöhte Stabilität bei der Lagerung.

Synthese der Ammoniumverbindungen III

Trimethyl-(4-(3)-aminobenzyl)-ammonium-methosulfat (Mischung)

Zu einer Lösung von 150 g (1 Mol) einer technischen Mischung von Dimethyl-(4- und 3-aminobenzyl)-amin in 1 000 ml Aceton läßt man bei 20-30 °C 126 g (1 Mol) Dimethylsulfat zutropfen. Man saugt das ausgefallene, farblose Reaktionsprodukt ab und erhält nach Trocknen im Vakuum ca. 262 g (95 % d. Th.) einer Mischung der Verbindungen der Formeln

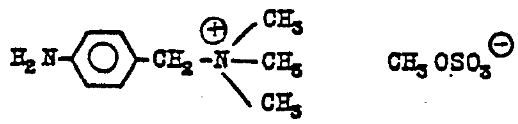

und

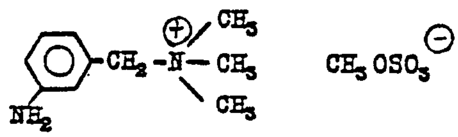

Trimethyl-(3-aminobenzyl)-ammonium-methosulfat

Zu einer Lösung von 150 g (1 Mol) Dimethyl-(3-aminobenzyl)-amin in 1 000 ml Aceton laßt man bei 20-30 °C 126 g (1 Mol) Dimethylsulfat zutropfen. Man saugt das ausgefallene, farblose Reaktionsprodukt ab und erhält nach Trocknen im Vakuum ca. 260 g (94 % d. Th.) der Verbindung der Formel

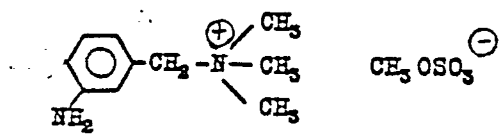

(Schmelzpunkt : 145 °C). Die Verbindung kann aus Isopropanol umkristallisiert werden.

Synthese der reaktiven Salze der Formel I

Beispiel 1

Zu einer Lösung von 276 g (1 Mol) Trimethyl-(4-(3)-aminobenzyl)-ammonium-methosulfat (Mischung) in 500 ml Wasser werden bei 0-5 °C 67,5 (0,5 Mol) 2,4,6-Trifluortriazin unter Rühren zugetropft. Durch Zugabe von Natronlauge, Sodalösung oder Bicarbonat wird ein pH-Wert von 5-7 eingehalten. Anschließend läßt man auf 20 °C erwärmen, rührt ca. 4 Stunden nach, wobei der pH-Wert mit Natronlauge, Sodalösung oder Bicarbonat bei 7 gehalten wird und filtriert gegebenenfalls. Man erhält eine Lösung, die ca. 300-320 g einer Mischung der Verbindungen der Formeln

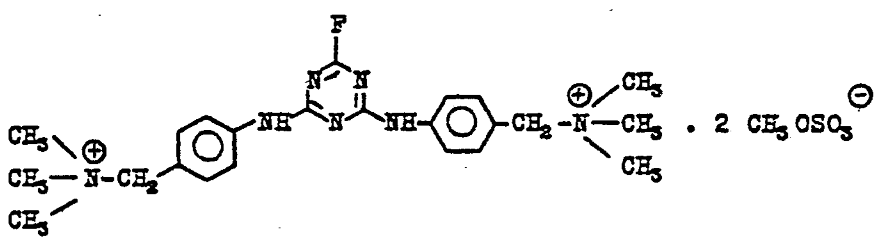

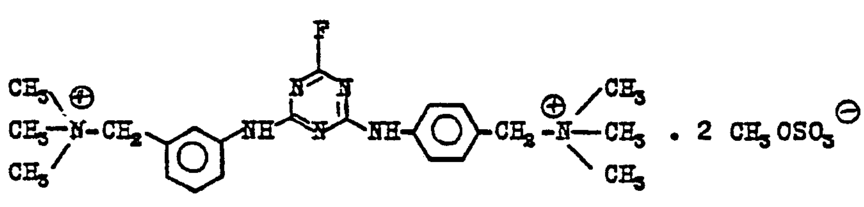

und

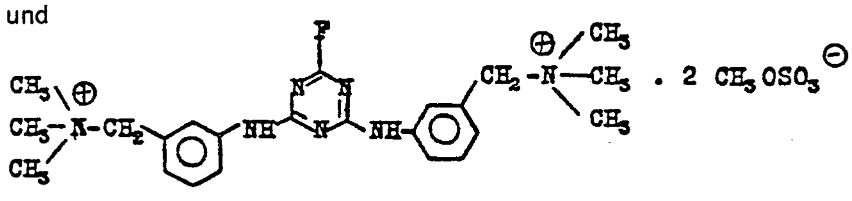

enthält. Man kann die wäßrige Lösung bei 20 °C im Vakuum einengen und erhält eine Paste, welche die Mischung dieser Verbindungen enthält ; man kann aber auch die wäßrige Lösung direkt verwenden.

Beispiel 2

Zu einer Lösung einer technischen Mischung von 150 g (1 Mol) Dimethyl-(4- und 3-aminobenzyl)-amin in 300 ml Wasser läßt man unter Rühren bei 0-20 °C 126 g (1 Mol) Dimethylsulfat zutropfen. Nach Abklingen der Wärmetönung rührt man noch 2 Stunden und versetzt anschließend bei 0-5 °C mit 67,5 g (0,5 Mol) 2,4,6-Trifluortriazin, wobei durch Zugabe von Natronlauge, Bicarbonat oder Sodalösung ein pH-Wert von 5-7 eingehalten wird. Anschließend läßt man auf 20 °C erwärmen, rührt ca. 4 Stunden nach, wobei der pH-Wert mit Natronlauge, Bicarbonat oder Sodalösung bei 7 gehalten wird und filtriert gegebenenfalls. Die erhaltene Lösung enthält ca. 300-320 g einer Mischung der Verbindungen der Formeln

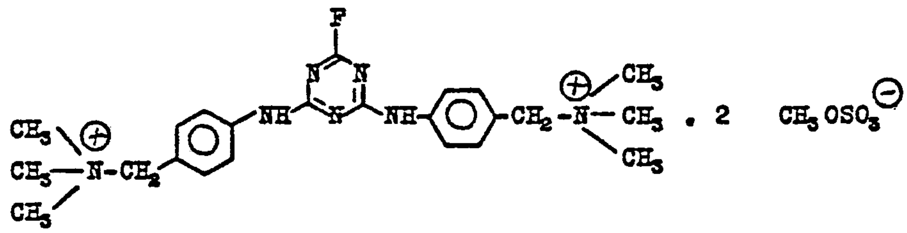

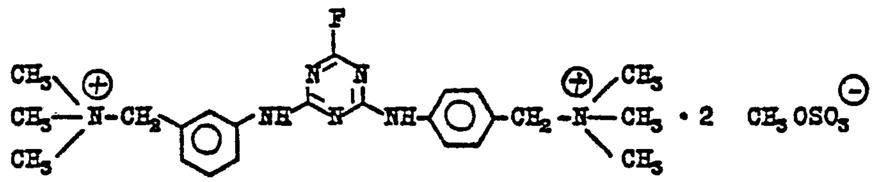

und

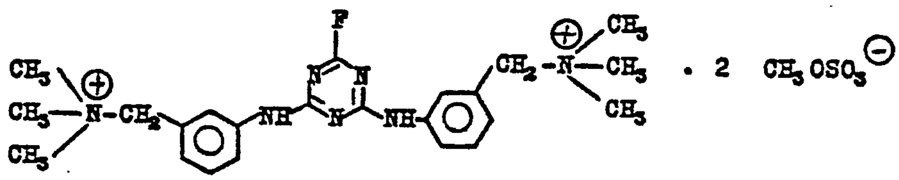

Die Lösung kann direkt in dieser Form verwendet werden.

Beispiel 3

Zu einer Lösung von 276 g (1 Mol) Trimethyl-(3-aminobenzyl)-ammonium-methosulfat in 500 ml Wasser werden bei 5-10 °C unter Rühren 92,25 g (0,5 Mol) Cyanurchlorid zugegeben. Durch Zugabe von Natronlauge, Bicarbonat oder Sodalösung wird ein pH-Wert von 5-7 eingehalten. Man läßt anschließend bei 20 °C bis zur klaren Lösung rähren, erwärmt dann auf 30 °C, wobei jeweils der pH-Wert mit Natronlauge, Bicarbonat oder Sodalösung bei 7 gehalten wird und filtriert gegebenenfalls. Man erhält eine Lösung, die ca. 300-330 g der Verbindung der Formel

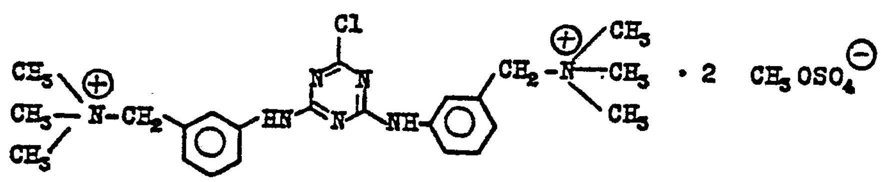

enthält. Diese Lösung kann man direkt verwenden, oder je nach Bedarf verdünnen, oder durch Abziehen von Wasser im Vakuum bei 20-30 °C aufkonzentrieren. Dasselbe gilt z. B. ebenso für die nach den Beispielen 1 und 2 erhaltenen Lösungen.

Man kann aber auch z. B. die nach diesem und nach den Beispielen 1 und 2 erhaltenen quartären Reaktiv-Verbindungen z. B. in der Weise isolieren, daß man das Wasser im Vakuum bei 20-30 °C abzieht, den Rückstand mit Methanol eluiert, von den ungelösten anorganischen Salzen abfiltriert und die methanolische Lösung einengt.

Einfacher und ökonomischer ist es hingegen, die erhaltene gegebenenfalls, wie oben beschrieben, verdünnte oder aufkonzentrierte wäßrige Lösung direkt zu verwenden.

In der folgenden Tabelle sind weitere Beispiele von Verbindungen der allgemeinen Formel I aufgeführt, die man erhalten kann, wenn man analog wie in den Beispielen 1 bis 3 verfährt.

| Bsp. Nr. | Verbindung III | Verbindung II | Verbindung I |
|---|---|---|---|
| 4 | 2 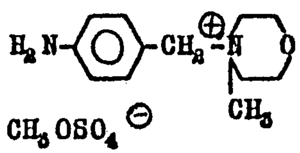 | 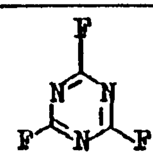 | 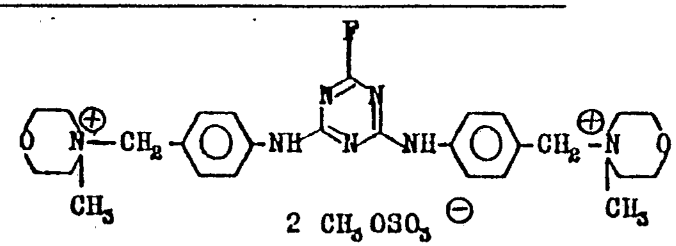 |
| 5 | 2 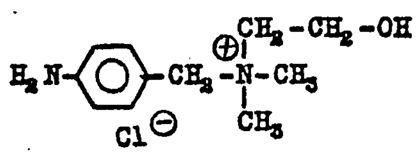 | 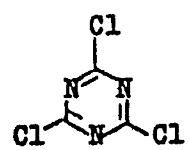 | 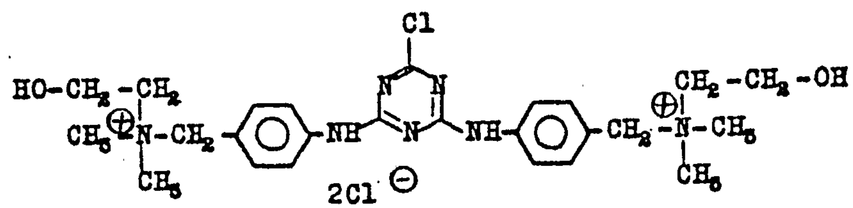 |

Beispiel 6

Auf einem Baumwollgewebeabschnitt wird eine Färbung von 3 % C.I. Direct Blue 225 (Colour Index (1971) 3. Aufl. Bd. 2) in der für diese Farbstoffe üblichen Färbeweise aus wäßriger Färbeflotte hergestellt, gespült und getrocknet. Auf einem Färbefoulard imprägniert man diese Färbung bei Zimmertemperatur mit einer Lösung, die im Liter

50 g der Verbindungen der Beispiele 1 bzw. 2 und
20 g Natriumcarbonat enthält,

und so abgepreßt wird, daß die Flottenaufnahme der Färbung etwa 80 % beträgt. Das Material wird sodann im Trockenschrank bei 60-70 °C getrocknet und anschließend 8 Minuten lang bei 102 °C gedämpft. Beim nun anschließenden Spülen bei Raumtemperatur und 60-70 °C während jeweils 5 Minuten bleiben die Spülbäder ungefärbt, beim darauffolgenden Behandeln im destillierten Wasser bei 95 °C erfolgt innerhalb 20 Minuten ein nur geringfügiges Anfärben der Behandlungsflotte.

Man erhält eine Blaufärbung mit sehr guten Licht-, Wasser-, Schweiß- und Waschechtheiten.

Eine Blaufärbung mit den genannten vorteilhaften Eigenschaften erhält man auch, wenn man als Farbstoff C.I. Direct Blue 151 ( = C.I. No. 24 175) verwendet.

Beispiel 7

Einen Baumwollgewebeabschnitt, der mit 3 % C.I. Direct Blue 225 gefärbt ist, behandelt man in einem frischen Bad, das — bezogen auf das Gewicht der Baumwolle —

4 % der Verbindungen der Beispiele 1 bzw. 2 sowie
2 ml/l 50 %ige Natriumhydroxidlösung enthält,

im Flottenverhältnis 1 : 30 bei 20-25 °C innerhalb von 25 Minuten. Dann spült man das Gewebe in kalten und 60 °C heißem Wasser aus und trocknet das Baumwollgewebe. Man erhält eine Blaufärbung mit sehr guter Licht-, Wasser-, Schweiß- und Waschechtheit.

Ähnliche Ergebnisse erhält man, wenn man anstelle von 4 % der Verbindungen der Beispiele 1 bzw. 2 4 % der Verbindung des Beispiels 4 verwendet.

Beispiel 8

Man stellt eine wäßrige Lösung her, die im Liter
60 g der Verbindung des Beispiels 3 und
30 g des Farbstoffs C.I. Direct Orange 76 ( = C.I. No. 40 270)
enthält.

Zu dieser Lösung gibt man 15 g Natriumcarbonat, imprägniert einen Baumwollgewebeabschnitt mit der Lösung und preßt ihn so ab, daß eine Flottenaufnahme von 80 % erfolgt. Das so behandelte Gewebe wird bei 60 °C getrocknet und 3 Minuten lang auf 150 °C erhitzt. Beim anschließenden Ausspülen in Wasser von Raumtemperatur und 60 °C während jeweils 5 Minuten und beim Behandeln in siedendem Wasser während 20 Minuten erfolgt kein Anfärben der Behandlungsflotten.

Die so erhaltene Scharlachfärbung zeichnet sich durch gute Licht-, Wasser-, Schweiß- und Waschechtheit aus.

Ähnliche Ergebnisse erhält man, wenn man anstelle von

60 g der Verbindung des Beispiels 3
70 g der Verbindung des Beispiels 4 verwendet.

**Ansprüche**

1. Verbindungen der Formel (I)

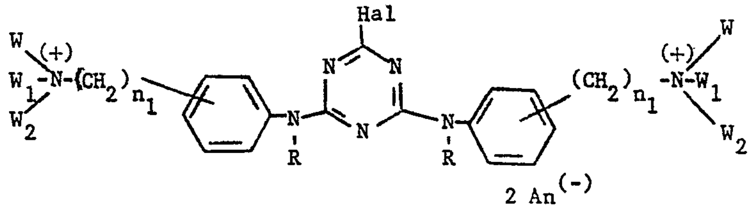

(I)

worin

Hal für Fluor oder Chlor,
R für Wasserstoff oder $C_1$-$C_4$-Alkyl,
$n_1$ für 1 oder 2,
W, $W_1$ und $W_2$ unabhängig voneinander für $C_1$-$C_6$-Alkyl, das durch

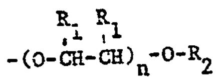

substituiert sein kann, wobei beide
$R_1$ für Wasserstoff stehen oder 1 $R_1$ für Wasserstoff und das andere für Methyl steht
$R_2$ Wasserstoff oder $C_1$-$C_4$-Alkyl ist und
n für 0 bis 4 steht
W außerdem für Benzyl oder
W und $W_2$ gemeinsam mit dem Stickstoffatom für Pyrrolidin, Piperidin, Morpholin oder Piperazin oder
W, $W_1$ und $W_2$ gemeinsam mit dem Stickstoffatom für Pyridin und
$An^{(-)}$ für ein Anion stehen.

2. Verfahren zur Herstellung von Verbindungen des Anspruchs 1, dadurch gekennzeichnet, daß man eine Verbindung der Formel (II)

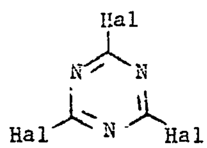
(II)

mit einer Verbindung der Formel (III)

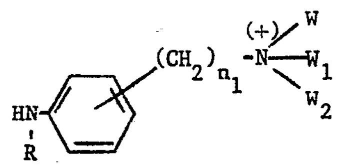 $An^{(-)}$
(III)

umsetzt, worin
Hal, R, W, $W_1$, $W_2$, $n_1$ und $An^{(-)}$ die in Anspruch 1 angegebene Bedeutung haben.

3. Verfahren zur Herstellung von Verbindungen des Anspruchs 1, dadurch gekennzeichnet, daß man eine Verbindung der Formel (IV)

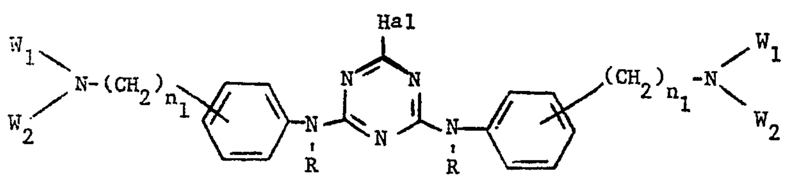
(IV)

a) mit einer Verbindung der Formel (V)

$$An'—W \qquad (V)$$

umsetzt, worin
R, $n_1$, W, $W_1$ und $W_2$ die in Anspruch 1 angegebene Bedeutung haben, und
An' für einen als Anion $An^{(-)}$ abspaltbaren Rest steht oder

b) zur Herstellung von Verbindungen mit W = 2-Hydroxyalkyl mit einer Verbindung der Formel (VI)

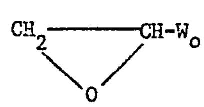
(VI)

in Gegenwart einer Säure
umsetzt, worin
R, $n_1$, $W_1$ und $W_2$ die in Anspruch 1 angegebene Bedeutung haben und
$W_0$ für Wasserstoff oder Alkyl, das 2 Kohlenstoffatome weniger als W hat, steht.

4. Verwendung von Verbindungen des Anspruchs 1 zur Erhöhung der Affinität von anionischen

Farbstoffen zu natürlichen oder synthetischen stickstoff- oder hydroxylgruppenhaltigen Fasern.

**Claims**

1. Compounds of the formula (I)

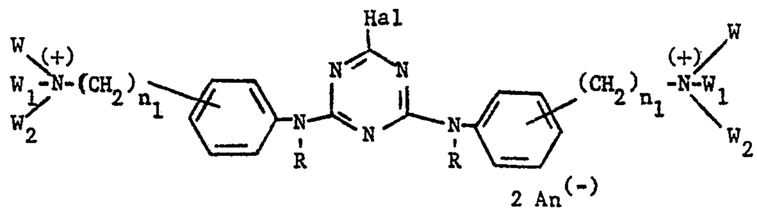

(I)

wherein

Hal represents fluorine or chlorine,

R represents hydrogen or $C_1$-$C_4$-alkyl,

$n_1$ represents 1 or 2,

W, $W_1$ and $W_2$ independently of one another represent $C_1$-$C_6$-alkyl, which can be substituted by

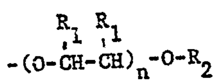

wherein

both $R_1$'s represent hydrogen or 1 $R_1$ represents hydrogen and the other represents methyl,

$R_2$ is hydrogen or $C_1$-$C_4$-alkyl and

n represents 0 to 4,

W also represents benzyl or

W and $W_2$, together with the nitrogen atom, represent pyrrolidine, piperidine, morpholine or piperazine or

W, $W_1$ and $W_2$, together with the nitrogen atom, represent pyridine and

$An^{(-)}$ represent an anion.

2. Process for the preparation of compounds of Claim 1, characterised in that a compound of the formula (II)

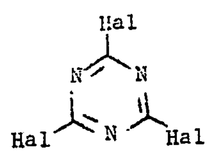

(II)

is reacted with a compound of the formula (III)

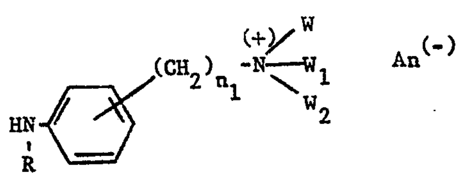

(III)

wherein

Hal, R, W, $W_1$, $W_2$, $n_1$ and $An^{(-)}$ have the meaning indicated in Claim 1.

3. Process for the preparation of compounds of Claim 1, characterised in that a compound of the formula (IV)

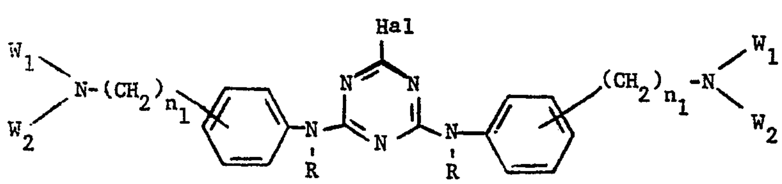

(IV)

is reacted

a) with a compound of the formula (V)

$An'-W$ (V)

wherein
R, $n_1$, W, $W_1$ and $W_2$ have the meaning indicated in Claim 1 and
An' represents a radical which can be split off as the anion $An^{(-)}$, or
b) for the preparation of compounds with W = 2-hydroxyalkyl, with a compound of the formula (VI)

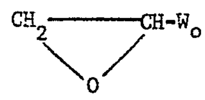
(VI)

in the presence of an acid, wherein
R, $n_1$, $W_1$ and $W_2$ have the meaning indicated in Claim 1, and
$W_0$ represents hydrogen or alkyl which has 2 carbon atoms fewer than W.

4. Use of compounds of Claim 1 for increasing the affinity of anionic dyestuffs for natural or synthetic fibres containing nitrogen or hydroxyl groups.

**Revendications**

1. Composés de formule (I)

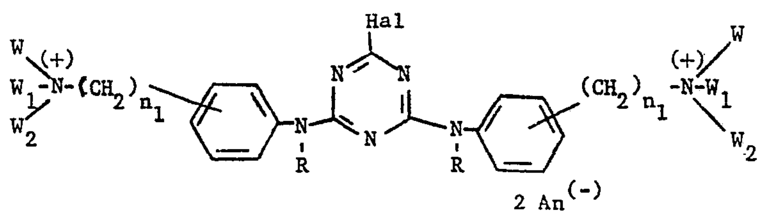
(I)

dans laquelle
Hal représente le fluor ou le chlore,
R est de l'hydrogène ou un groupe alkyle en $C_1$-$C_4$,
$n_1$ vaut 1 ou 2,
W, $W_1$ et $W_2$ représentent chacun, indépendamment l'un de l'autre, un groupe alkyle en $C_1$-$C_6$, qui peut être substitué par

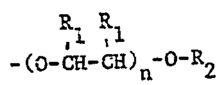

dans laquelle
les deux $R_1$ représentent de l'hydrogène ou bien un $R_1$ est de l'hydrogène et l'autre est un groupe méthyle,
$R_2$ est de l'hydrogène ou un groupe alkyle en $C_1$-$C_4$, et
n vaut 0 à 4,
W peut en outre représenter un groupe benzyle, ou bien
W et $W_2$, pris avec l'atome d'azote, peuvent représenter la pyrrolidine, la pipéridine, la morpholine ou la pipérazine, ou bien
W, $W_1$ et $W_2$, pris avec l'atome d'azote, représentent de la pyridine, et
$An^{(-)}$ est un anion.

2. Procédé de production de composés de la revendication 1, caractérisé en ce qu'on fait réagir un composé de formule (II)

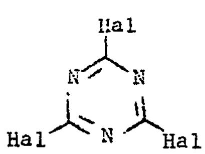
(II)

avec un composé de formule (III)

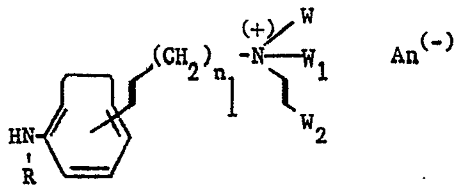

(III)

où

Hal, R, W, $W_1$, $W_2$, $n_1$ et $An^{(-)}$ ont le sens indiqué à la revendication 1.

3. Procédé de production de composés de la revendication 1, caractérisé en ce qu'on fait réagir un composé de formule (IV)

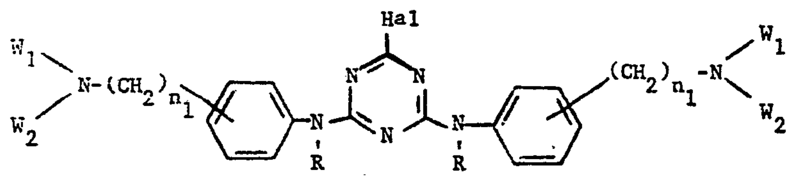

(IV)

a) avec un composé de formule (V)

$$An'—W \qquad (V)$$

où

R, $n_1$, W, $W_1$ et $W_2$ ont le sens indiqué à la revendication 1, et

An' représente un radical scindable sous forme d'un anion $An^{(-)}$, ou

b) pour produire des composés dans lesquels W est un groupe 2-hydroxyalkyle, avec un composé de formule (VI)

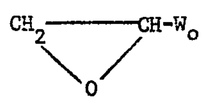

(VI)

en présence d'un acide, où

R, $n_1$, $W_1$ et $W_2$ ont le sens indiqué à la revendication 1, et

$W_0$ est de l'hydrogène ou un groupe alkyle comportant deux atomes de carbone de moins que W.

4. Utilisation des composés de la revendication 1, pour élever l'affinité de colorants anioniques pour des fibres naturelles ou synthétiques contenant de l'azote ou des groupes hydroxyles.